# EUROPEAN PATENT APPLICATION

(11) **EP 4 803 069 A1**
(43) Date of publication of application: **09.09.2026**
(21) Application number: 24884794.9
(22) Date of filing: 30.10.2024
(51) Int. Cl.: A61K 8/34, A61K 9/107, A61Q 19/08, A61P 21/02

(54) **MACROMOLECULAR TRANSDERMAL MICROEMULSION SYSTEM AND USE THEREOF**

(30) Priority: 31.10.2023 CN 202311439455
(71) Applicant: YSTE (Hainan) Aesthetic Medicine Health Technology Co., Ltd., Sanya, Hainan 572025 (CN); Kangma-Healthcode (Shanghai) Biotech Co., Ltd, Pudong New Area Shanghai 201321 (CN)
(72) Inventor: GUO, Min, Shanghai 201321 (CN); JIANG, Zhuyan, Shanghai 201321 (CN); LIU, Zhang, Shanghai 201321 (CN); XIAO, Song, Shanghai 201321 (CN); YU, Xue, Shanghai 201321 (CN)
(74) Representative: Dai, Simin
(86) International application number: PCT/CN2024/128410
(87) International publication number: WO 2025/092792

(57) **Abstract**

A macromolecular transdermal microemulsion system capable of enabling a macromolecular active substance to effectively transdermally exert a predetermined effect and use thereof. The macromolecular transdermal microemulsion system comprises an effective amount of a macromolecular active substance capable of exerting a predetermined effect. The macromolecular transdermal microemulsion system comprises: 7%-36% by mass of an oil phase, 57%-88% by mass of a water phase, and 8%-20% by mass of an emulsifier.

## Description

### TECHNICAL FIELD

The present invention belongs to the field of biotechnology, specifically relating to a macromolecular transdermal microemulsion system and use thereof.

### BACKGROUND

The transdermal system is a method of drug absorption through the skin, wherein the drug is absorbed through the skin into the human blood circulation to reach an effective blood drug concentration, thereby achieving disease treatment or prevention, which is a new route of administration.

The transdermal drug delivery system has many advantages: it can effectively reduce the pain of patients during injection administration, effectively avoid the irritation and side effects caused by oral drugs, effectively improve the sustained-release effect of drugs, and enhance the safety of drug use and treatment. Transdermal administration has many advantages such as avoiding hepatic first-pass effect, more stable blood drug concentration, and high safety.

However, human skin mainly functions as a barrier, wherein this structure is conducive to "internal stability and external defense," protecting the human body from external stimuli, while also preventing the entry of external components; so that for external components to enter the skin, they must first pass through the epidermal layer of the skin and enter the interior of the skin to exert effects.

The skin is composed of three parts: epidermis, dermis, and subcutaneous tissue. Among them, the epidermis is the shallowest layer of the skin; the epidermis is divided into stratum corneum, stratum lucidum, stratum granulosum, stratum spinosum, and stratum basale; the stratum basale is connected to the dermis by the basement membrane. The stratum corneum is the outermost layer of the epidermis, composed of 5 to 15 layers of keratinocytes and intercellular lipid; this structure is vividly compared to a "brick wall structure". The thickness of the stratum corneum is 15 to 50 µm, has very low water content (5% to 20%) and inactive metabolism, and is the main barrier for chemical substance transdermal absorption. Intercellular lipid mainly composed of 45% to 50% ceramide, 25% cholesterol, 15% long-chain free fatty acid, and 5% other lipids. The dermis is located below the epidermis and supports the epidermis; the dermis is mainly composed of connective tissue, including collagen fiber, elastic fiber, and matrix. The dermis also contains other tissues such as nerves, blood vessels, lymphatic vessels, muscles, hair follicles, sebaceous glands, and large and small sweat glands.

Due to the dense stratum corneum structure of the skin, most drugs cannot be absorbed into the systemic circulation, and bioavailability does not meet clinical needs; especially for macromolecular bioactive drugs, it is more difficult to penetrate the skin to exert efficacy. Therefore, improving the transdermal permeation of drugs is the key and difficulty in the research and development of transdermal drug formulations.

Commonly used physical transdermal permeation enhancement technologies include: microneedle, iontophoresis, electroporation technology, ultrasound introduction technology, and needle-free drug delivery system; commonly used chemical penetration enhancers chemically include transdermal permeation enhancers and ion pairs.

A transdermal penetration enhancer is a substance capable of promoting drug transdermal absorption, which is a preferred method. Currently, transdermal drug delivery systems have rapidly developed in drug development, with new products continuously entering the market, such as estradiol, testosterone and other drugs. In the beauty and medical aesthetics industry, some high-end cosmetic products are also being developed as mulsion-type formulations to achieve superior efficacy.

However, there is still a lack of better products for macromolecular transdermal delivery.

### SUMMARY

The present invention provides a macromolecular transdermal microemulsion system capable of allowing macromolecular active substances to effectively perform predetermined actions transdermally, and use thereof.

To this end, the present invention provides the following technical solutions.

The present invention provides a macromolecular transdermal microemulsion system, comprising an effective amount of macromolecular active substances capable of performing predetermined actions; by the mass percentage based on the total weight of the transdermal microemulsion system, the composition of the macromolecular transdermal microemulsion system is: an oil phase: 7-36%; an aqueous phase: 57%-88%; an emulsifier: 8%-20%.

The macromolecular transdermal microemulsion system provided by the present invention further has the feature that: the oil phase accounts for 20%-30%, the aqueous phase accounts for 60%-70%, and the emulsifier accounts for 8%-12%; and/or based on the mass ratio, the composition of the transdermal microemulsion system is: the oil phase: the aqueous phase: the emulsifier: 2-2.5 : 6-7 : 1.

The macromolecular transdermal microemulsion system provided by the present invention further has the feature that: the macromolecular active substance is less than or equal to 300 kDa or less than or equal to 150 kD, and/or by the mass percentage based on the total weight of the transdermal microemulsion system, the effective amount of the macromolecular active substance in the transdermal microemulsion system is: the proportion of the macromolecular active substance is less than or equal to 10%, or less than or equal to 5%, or less than or equal to 2%, or less than or equal to 1%, preferably, the macromolecular active substance is a biomolecule capable of providing therapeutic, cosmetic, and skin care effects; further preferably, the macromolecular active substance is water-soluble.

The macromolecular transdermal microemulsion system provided by the present invention further has the feature that: wherein the contained macromolecular active substance is at least selected from any one or more of clostridial neurotoxin, BSA, soluble collagen, and elastin, and/or provided by yeast extract contained in the system, preferably, the clostridial neurotoxin is botulinum toxin of type A, B, C, D, E, F, or G, and/or the light chain of the clostridial neurotoxin contains an amino acid sequence having at least 35%, 50%, 60%, 70%, 75%, 80%, 85%, 90%, 99%, or 100% identity with any one of SEQ ID NOs: 1-7; and/or the heavy chain of the clostridial neurotoxin contains an amino acid sequence having at least 35%, 50%, 60%, 70%, 75%, 80%, 85%, 90%, 99%, or 100% identity with any one of SEQ ID NOs: 8-14.

The macromolecular transdermal microemulsion system provided by the present invention further has the feature that: by the mass percentage based on the total weight of the transdermal microemulsion system, in the system: the effective amount of the clostridial neurotoxin is less than or equal to 0.02%, preferably 0.00001%-0.015%, more preferably 0.0005%-0.015% or 0.0008%-0.0015%; the effective amounts of the soluble collagen, BSA, and the elastin are each less than or equal to 5%, or less than or equal to 2%, or less than or equal to 1%; the yeast extract accounts for 1%-10%.

The macromolecular transdermal microemulsion system provided by the present invention further has the feature that: Based on the mass percentage of the total weight of the transdermal microemulsion system, the composition of the system comprises: 5%-20% or 10%-20% or 13%-18% of glycerol; 5%-20% or 5%-15% or 8%-12% of emulsifier, 1%-10% or 3%-8% of butylene glycol or propylene glycol, 0.5%-5% or 1%-3% of laurocapram, 0.3%-3% or 1%-2% of carboxymethyl deacetylated chitin or chitosan derivative, 55%-80% or 60%-70% of water, and the effective amount of the macromolecular active substance.

The macromolecular transdermal microemulsion system provided by the present invention further has the feature that: the emulsifier is selected from one or more of polysorbate 80, polysorbate 60, polysorbate 20, and sorbitan oleate.

The macromolecular transdermal microemulsion system provided by the present invention further has the feature that: the macromolecular transdermal microemulsion system contains a preservative, preferably, by the mass percentage based on the total weight of the transdermal microemulsion system: the macromolecular transdermal microemulsion system contains a preservative in a mass percentage of 0.1%-1%, and further, the preservative is selected from any one or more of phenoxyethanol, methylparaben, ethylparaben, propylparaben, benzyl alcohol, and sorbic acid.

The macromolecular transdermal microemulsion system provided by the present invention further has the feature that: a particle size of particles of the transdermal microemulsion system is on average 10-15 nm.

The present invention further provides use of the macromolecular transdermal microemulsion system in achieving therapeutic, cosmetic, and skincare effects.

The use provided by the present invention above is characterized in that, when the macromolecular transdermal microemulsion system is used, transdermal administration is performed by any one or more of topical application, transdermal instrument, patch application, and spray.

The present invention further provides use of a macromolecular transdermal microemulsion system in the manufacture of a medicament or a product for achieving therapeutic, cosmetic, and skincare effects.

### EFFECTS AND FUNCTIONS OF THE INVENTION

The macromolecular transdermal microemulsion system and use thereof provided by the present invention, due to the appropriate proportion of the oil phase, aqueous phase, and emulsifier in the composition of the macromolecular transdermal microemulsion system, can form an oil-in-water microemulsion with an average particle size at the nanoscale, such that the macromolecular active substance is effectively absorbed transdermally to achieve the predetermined effect.

### BRIEF DESCRIPTION OF THE DRAWINGS

FIG. 1 shows a standard curve of BSA standard according to four-parameter fitting in example 4;
FIG. 2 shows a toe syndactyly phenotype diagram of mouse experiments in example 5.

### DETAILED DESCRIPTION OF THE PREFERRED EMBODIMENT

The detailed embodiments of the present invention are described below in conjunction with the drawings. For the specific methods or materials used in the embodiments, those skilled in the art can make conventional substitutions and selections based on the technical concept of the present invention according to existing technologies, and the specific methods or materials used in the embodiments are not limited to the specific descriptions of the embodiments of the present invention.

Unless otherwise specified, the methods used in the embodiments are conventional methods; unless otherwise specified, the materials, reagents, etc. used can be obtained from commercial sources.

Emulsion refers to a non-uniform dispersed liquid preparation formed by two immiscible liquid phases, wherein one phase is dispersed in the other phase in the form of small droplets. The phase forming the droplets is called the dispersed phase, internal phase, or discontinuous phase, and the other liquid phase is called the dispersion medium, external phase, or continuous phase.

The emulsion is composed of an oil phase (denoted as O), an emulsifier, and an aqueous phase (denoted as W). According to the type of emulsifier, properties, and phase volume ratio (φ), an oil-in-water (O/W) emulsion, a water-in-oil (W/O) emulsion, or a multiple emulsion can be formed.

Aqueous phase: water or aqueous solution. Aqueous solution refers to a phase in which raw materials soluble in water are dissolved in water;
oil phase: it refers to a phase in which raw materials poorly soluble in water are dissolved in oily substances.

According to the particle size of the emulsion particles, that is, the size of the emulsion droplets, the emulsion is classified as an ordinary emulsion, a submicroemulsion, and a nanoemulsion:
1. Ordinary emulsion: The droplet size of the ordinary emulsion is generally 1 µm-100 µm, presenting a milky white opaque liquid.
2. Submicroemulsion: The droplet size is generally 0.1 µm-1.0 µm, and submicroemulsions are often used as carriers for gastrointestinal administration. Emulsions for intravenous injection should be submicroemulsions, with particle sizes generally in the range of 0.25 µm-0.4 µm.
3. Nanoemulsion: The droplet size is < 100 nm, generally in the range of 10-100 nm, and nanoemulsions are also called microemulsions.

The macromolecular transdermal microemulsion system provided by the present invention comprises an effective amount of a macromolecular active substance capable of exerting a predetermined effect; by the mass percentage based on the total weight of the transdermal microemulsion system, the composition of the macromolecular transdermal microemulsion system is: an oil phase: 16%-36% or 20%-30%; an aqueous phase: 55%-88% or 60%-70%; an emulsifier: 8%-20% or 10%-25%.

The macromolecular active substance refers to substances with larger molecular weight, mainly referring to biomolecules.

Dalton (Da) is the full name of the unit Dalton, which is a common unit of molecular weight, defined as the algebraic sum of the atomic weights of all atoms in a molecule according to their number. Dalton (Da) is numerically equal to the relative molecular mass.

In biochemistry, molecular biology, and proteomics, kDa (kilodalton) is often used to represent biological macromolecules such as proteins. 1 kDa represents a molecule with a relative molecular mass of 1000; macromolecules refer to biological substances with a relative molecular mass above 5000, even exceeding one million, such as polypeptides, proteins, nucleic acids, polysaccharides, and antibodies.

The composition of the macromolecular transdermal microemulsion system provided by the present invention is shown in Table 1:

| Table 1 | |
|---|---|
| Based on the mass percentage of the total weight of the macromolecular transdermal microemulsion system | |
| Oil phase | 7%-36% |
| Aqueous phase | 57%-88% |
| Emulsifier | 8%-20% |

In Table 1, it refers to the composition of the macromolecular transdermal microemulsion system of the present invention, which is divided into three types: oil phase, aqueous phase, and emulsifier, wherein the "effective amount of macromolecular active substance capable of exerting a predetermined effect" may be oily (soluble in oil) or aqueous (soluble in water).

In one example, the macromolecular active substance of the present invention refers to a biomolecule less than or equal to approximately 300 kDa or less than or equal to approximately 150 kDa, preferably, the biomolecule is water-soluble, such that after addition, it exists in the aqueous phase.

In one example, the predetermined effect is any one or more effects selected from therapeutic, cosmetic, and skin care effects.

In one example, the limitation of the macromolecular transdermal microemulsion system of the present invention satisfies any one or two of those shown in Table 2:

| Table 2 |
|---|
| First type: Based on the mass percentage of the total weight of the macromolecular transdermal system: Oil phase: 20%-30%; Aqueous phase: 60%-70%; Emulsifier 8%-12% |
| Second type: based on the mass ratio, the composition of the transdermal microemulsion system is: oil phase: Aqueous phase: the emulsifier: 2-2.5 : 6-7 : 1 |

The macromolecular active substance can exert its effect through the system. Based on the mass percentage of the total weight of the transdermal microemulsion system, the effective amount of the macromolecular active substance in the transdermal microemulsion system is: the proportion of the macromolecular active substance is less than or equal to 10%, or less than or equal to 5%, or less than or equal to 2%, or less than or equal to 1%. Within this range, according to the specific macromolecular active substance, adjustments are made, and after the final adjustment, the overall oil phase, aqueous phase, and emulsifier of the microemulsion system meet the aforementioned ratio or proportion requirements.

In one example, the contained macromolecular active substance is at least selected from any one or more of clostridial neurotoxin, soluble collagen, and elastin, and/or provided by yeast extract contained in the system: that is, the contained macromolecular active substance may have several situations as shown in Table 3:

| Table 3 Types or sources of contained macromolecular active substances | |
|---|---|
| First type | Selected only from any one or more of clostridial neurotoxin, soluble collagen, and elastin |
| Second type | Excluding the first type, the transdermal microemulsion system includes yeast extract, wherein the macromolecular active substance comes from components in the yeast extract. |
| Third type | Different selections in the first type in combination with the second type |

In one example, for any case in Table 3, by the mass percentage based on the total weight of the transdermal microemulsion system, the content is as follows:
the effective amount of the clostridial neurotoxin is less than or equal to 0.02%, preferably 0.00001%-0.015%, more preferably 0.0005%-0.015% or 0.0008%-0.0015%; the content of the clostridial neurotoxin is 10 ng/mL-10 µg/mL;

The effective amounts of the soluble collagen and the elastin are both less than or equal to 5%, or less than or equal to 2%, or less than or equal to 1% by proportion;

The effective amount is provided by the yeast extract contained in the emulsion system at a proportion of 1%-10%, for example, if the yeast extract accounts for 10%, and the macromolecule contained accounts for 5%, then the effective amount is 5%.

The clostridial neurotoxin can be used for treatment and cosmetic purposes. For example, botulinum toxin can interfere with the exocytosis of presynaptic membrane vesicles, inhibit the release of acetylcholine (Ach) from nerve terminals, cause chemical denervation of muscles, and thus lead to muscle relaxation and relieving muscle spasm to exert therapeutic effects by specifically binding to the presynaptic membrane receptors of peripheral cholinergic nerve terminals and cleaving the synapse-associated protein SNAP-25. The chemical denervation effect of the botulinum toxin on muscles is the theoretical basis for regulating hypertonia, movement disorder disease, and dynamic wrinkles; Furthermore, multiple injections into the target muscle can cause disuse muscle atrophy and reduce muscle volume, therefore the botulinum toxin is also used for body contouring. Currently, the applications of the botulinum toxin in the field of disease treatment include: hemifacial spasm, idiopathic blepharospasm, spasmodic torticollis, spastic cerebral palsy, post-stroke limb spasticity, tremor, and other various muscle tone disorders. The applications of the botulinum toxin in the minimally invasive plastic surgery field include wrinkle removal of various dynamic wrinkles such as glabellar lines, forehead lines, and crow's feet; adjustment of eyebrow height, mentalis muscle relaxation, jawline lift, platysmal band injection, and other facial contour beautifications; as well as body contouring by reducing the masseter muscle, reducing the gastrocnemius muscle, and reducing the trapezius muscle.

The clostridial neurotoxin in this document refers to natural clostridial neurotoxin and toxins having similar structures and functions to natural clostridial neurotoxin.

Although the amino acid sequences and immunogenicity of various types of toxins differ, they all exhibit similar molecular structures. Clostridial neurotoxin is produced by toxin-producing clostridia in the form of a non-toxic single-chain polypeptide, approximately 150 kD. Only after cleavage by bacterial protease or in vitro protease into a double-chain form does it become active, that is, composed of a light chain (L chain, amino terminus of the toxin, 50 kD) and a heavy chain (H chain, carboxyl terminus of the toxin, 100 kD) connected by a disulfide bond. The heavy chain is further composed of two domains, Hn (amino terminus, 50 kD) and Hc (carboxyl terminus, 50 kD).

Furthermore, the clostridial neurotoxin is botulinum toxin of type A, B, C, D, E, F, or G, and/or the light chain of the clostridial neurotoxin contains an amino acid sequence having at least 35%, 50%, 60%, 70%, 75%, 80%, 85%, 90%, 99%, or 100% identity with any one of SEQ ID NOs: 1-7; and/or the heavy chain of the clostridial neurotoxin contains an amino acid sequence having at least 35%, 50%, 60%, 70%, 75%, 80%, 85%, 90%, 99%, or 100% identity with any one of SEQ ID NOs: 8-14. The sequences are shown in Table 4.

| Table 4 Sequence listing | |
|---|---|
| SEQ ID NO: 1 | MPFVNKQFNYKDPVNGVDIAYIKIPNVGQMQPVKAFKIHNKIWVIPER DTFTNPEEGDLNPPPEAKQVPVSYYDSTYLSTDNEKDNYLKGVTKLF ERIYSTDLGRMLLTSIVRGIPFWGGSTIDTELKVIDTNCINVIQPDGSYR SEELNLVIIGPSADIIQFECKSFGHEVLNLTRNGYGSTQYIRFSPDFTFGF EESLEVDTNPLLGAGKFATDPAVTLAHELIHAGHRLYGIAINPNRVFKV NTNAYYEMSGLEVSFEELRTFGGHDAKFIDSLQENEFRLYYYNKFKDI ASTLNKAKSIVGTTASLQYMKNVFKEKYLLSEDTSGKFSVDKLKFDK LYKMLTEIYTEDNFVKFFKVLNRKTYLNFDKAVFKINIVPKVNYTIYD GFNLRNTNLAANFNGQNTEINNMNFTKLKNFTGLFEFYKLLCVRGIIT SQTQS |
| SEQ ID NO: 2 | MPVTINNFNYNDPIDNNNIIMMEPPFARGTGRYYKAFKITDRIWIIPER YTFGYKPEDFNKSSGIFNRDVCEYYDPDYLNTNDKKNIFLQTMIKLFN RIKSKPLGEKLLEMIINGIPYLGDRRVPLEEFNTNIASVTVNKLISNPGE VERKKGIFANLIIFGPGPVLNENETIDIGIQNHFASREGFGGIMQMKFCP EYVSVFNNVQENKGASIFNRRGYFSDPALILMHELIHVLHGLYGIKVD DLPIVPNEKKFFMQSTDAIQAEELYTFGGQDPSIITPSTDKSIYDKVLQN FRGIVDRLNKVLVCISDPNININIYKNKFKDKYKFVEDSEGKYSIDVES FDKLYKSLMFGFTETNIAENYKIKTRASYFSDSLPPVKIKNLLDNEIYTI EEGFNISDKNMEKEYRGQNKAINKQAYEEISKEHLAVYKIQMCKSVR |
| SEQ ID NO: 3 | MPITINNFNYSDPVDNKNILYLDTHLNTLANEPEKAFRITGNIWVIPDR FSRNSNPNLNKPPRVTSPKSGYYDPNYLSTDSDKDTFLKEIIKLFKRINS REIGEELIYRLSTDIPFPGNNNTPINTFDFDVDFNSVDVKTRQGNNWV KTGSINPSVIITGPRENIIDPETSTFKLTNNTFAAQEGFGALSIISISPRFM LTYSNATNDVGEGRFSKSEFCMDPILILMHELNHAMHNLYGIAIPNDQ TISSVTSNIFYSQYNVKLEYAEIYAFGGPTIDLIPKSARKYFEEKALDYY RSIAKRLNSITTANPSSFNKYIGEYKQKLIRKYRFVVESSGEVTVNRNK FVELYNELTQIFTEFNYAKIYNVQNRKIYLSNVYTPVTANILDDNVYDI QNGFNIPKSNLNVLFMGQNLSRNPALRKVNPENMLYLFTKFCHKAID GR |
| SEQ ID NO: 4 | MTWPVKDFNYSDPVNDNDILYLRIPQNKLITTPVKAFMITQNIWVIPE |
| | RFSSDTNPSLSKPPRPTSKYQSYYDPSYLSTDEQKDTFLKGIIKLFKRIN ERDIGKKLINYLVVGSPFMGDSSTPEDTFDFTRHTTNIAVEKFENGSW KVTNIITPSVLIFGPLPNILDYTASLTLQGQQSNPSFEGFGTLSILKVAPE FLLTFSDVTSNQSSAVLGKSIFCMDPVIALMHELTHSLHQLYGINIPSDK RIRPQVSEGFFSQDGPNVQFEELYTFGGLDVEIIPQIERSQLREKALGH YKDIAKRLNNINKTIPSSWISNIDKYKKIFSEKYNFDKDNTGNFVVNID KFNSLYSDLTNVMSEVVYSSQYNVKNRTHYFSRHYLPVFANILDDNIY TIRDGFNLTNKGFNIENSGQNIERNPALQKLSSESVVDLFTKVCLRLTK NSR |
| SEQ ID NO: 5 | MPTINSFNYNDPVNNRTILYIKPGGCQQFYKSFNIMKNIWIIPERNVIGT IPQDFLPPTSLKNGDSSYYDPNYLQSDQEKDKFLKIVTKIFNRINDNLS GRILLEELSKANPYLGNDNTPDGDFIINDASAVPIQFSNGSQSILLPNVII MGAEPDLFETNSSNISLRNNYMPSNHGFGSIAIVTFSPEYSFRFKDNSM NEFIQDPALTLMHELIHSLHGLYGAKGITTKYTITQKQNPLITNIRGTNI EEFLTFGGTDLNIITSAQSNDIYTNLLADYKKIASKLSKVQVSNPLLNP YKDVFEAKYGLDKDASGIYSVNINKFNDIFKKLYSFTEFDLATKFQVK CRQTYIGQYKYFKLSNLLNDSIYNISEGYNINNLKVNFRGQNANLNPR IITPITGRGLVKKIIRFCKNIVSVKGIR |
| SEQ ID NO: 6 | MPVAINSFNYNDPVNDDTILYMQIPYEEKSKKYYKAFEIMRNVWIIPE RNTIGTNPSDFDPPASLKNGSSAYYDPNYLTTDAEKDRYLKTTIKLFKR INSNPAGKVLLQEISYAKPYLGNDHTPIDEFSPVTRTTSVNIKLSTNVES SMLLNLLVLGAGPDIFESCCYPVRKLIDPDVVYDPSNYGFGSINIVTFS PEYEYTFNDISGGHNSSTESFIADPAISLAHELIHALHGLYGARGVTYE ETIEVKQAPLMIAEKPIRLEEFLTFGGQDLNIITSAMKEKIYNNLLANY EKIATRLSEVNSAPPEYDINEYKDYFQWKYGLDKNADGSYTVNENKF NEIYKKLYSFTESDLANKFKVKCRNTYFIKYEFLKVPNLLDDDIYTVSE GFNIGNLAVNNRGQSIKLNPKIIDSIPDKGLVEKIVKFCKSVIPRKGTK |
| SEQ ID NO: 7 | MPVNIKXFNYNDPINNDDIIMMEPFNDPGPGTYYKAFRIIDRIWIVPER FTYGFQPDQFNASTGVFSKDVYEYYDPTYLKTDAEKDKFLKTMIKLF NRINSKPSGQRLLDMIVDAIPYLGNASTPPDKFAANVANVSINKKIIQP GAEDQIKGLMTNLIIFGPGPVLSDNFTDSMIMNGHSPISEGFGARMMIR FCPSCLNVFNNVQENKDTSIFSRRAYFADPALTLMHELIHVLHGLYGIK ISNLPITPNTKEFFMQHSDPVQAEELYTFGGHDPSVISPSTDMNIYNKA LQNFQDIANRLNIVSSAQGSGIDISLYKQIYKNKYDFVEDPNGKYSVD KDKFDKLYKALMFGFTETNLAGEYGIKTRYSYFSEYLPPIKTEKLLDN TIYTQNEGFNIASKNLKTEFNGQNKAVNKEAYEEISLEHLVIYRIAMCK PVMYKNTGK |
| SEQ ID NO: 8 | ALNDLCIKVNNWDLFFSPSEDNFTNDLNKGEEITSDTNIEAAEENISLD LIQQYYLTFNFDNEPENISIENLSSDIIGQLELMPNIERFPNGKKYELDK YTMFHYLRAQEFEHGKSRIALTNSVNEALLNPSRVYTFFSSDYVKKVN KATEAAMFLGWVEQLVYDFTDETSEVSTTDKIADITIIIPYIGPALNIGN MLYKDDFVGALIFSGAVILLEFIPEIAIPVLGTFALVSYIANKVLTVQTID NALSKRNEKWDEVYKYIVTNWLAKVNTQIDLIRKKMKEALENQAEA TKAIINYQYNQYTEEEKNNINFNIDDLSSKLNESINKAMININKFLNQC |
| | SVSYLMNSMIPYGVKRLEDFDASLKDALLKYIYDNRGTLIGQVDRLK DKVNNTLSTDIPFQLSKYVDNQRLLSTFTEYINNIINTSILNLRYESNHL IDLSRYASKINIGSKVNFDPIDKNQIQLFNLESSKIEVILKNAIVYNSMY ENFSTSFWIRIPKYFNSISLNNEYTIINCMENNSGWKVSLNYGEIIWTLQ DTQEIKQRVVFKYSQMINISDYINRWIFVTITNNRLNNSKIYINGRLIDQ KPISNLGNIHASNNIMFKLDGCRDTHRYIWIKYFNLFDKELNEKEIKDL YDNQSNSGILKDFWGDYLQYDKPYYMLNLYDPNKYVDVNNVGIRG YMYLKGPRGSVMTTNIYLNSSLYRGTKFIIKKYASGNKDNIVRNNDRV YINVVVKNKEYRLATNASQAGVEKILSALEIPDVGNLSQVVVMKSKN DQGITNKCKMNLQDNNGNDIGFIGFHQFNNIAKLVASNWYNRQIERSS RTLGCSWEFIPVDDGWGERPL |
| SEQ ID NO: 9 | APGICIDVDNEDLFFIADKNSFSDDLSKNERIEYDTQSNYIENRSSINELI LDTNLISKIELPSENTESLTDFNVDVPVYEKQPAIKKIFTDENTIFQYLYS QTFPLDIRDISLTSSFDDALLFSNKVYSFFSMDYIKTANKVVEAGLFAG WVKQIVDDFVIEANKSSTMDKIADISLIVPYIGLALNVGNETAKGNFE NAFEIAGASILLEFIPELLIPVVGAFLLESYIDNKNKIIKTIDNALTKRDE KWIDMYGLIVAQWLSTVNTQFYTIKEGMYKALNYQAQALEEIIKYKY NIYSEKEKSNINIDFNDINSKLNEGINQAIDNINDFINECSVSYLMKKMI PLAVEKLLDFDNTLKKNLLNYIDENKLYLIGSAEYEKSKVDKYLKTIIP FDLSMYTNNTILIEIFNKYNSEILNNIILNLRYRDNNLIDLSGYGANVEV YDGVKLNDKNQFKLTSSTNSEIRVTQNQNIIFNSMFLDFSVSFWIRIPK YKNDGIQNYIHNEYTIINCIKNNSGWKISIRGNRIIWTLTDINGKTKSVF FEYSIREDISDYINRWFFVTITNNSDNAKIYINGKLESNIDIKNIGEVIAN GEIIFKLDGDIDRTQFIWMKYFSIFNTELSQSNIKEIYKIQSYSEYLKDF WGNPLMYNKEYYMFNAGNKNSYIKLKKDSSVGEILTRSKYNQNSNY INYRNLYIGEKFIIRRKSNSQSINDDIVRKEDYIYLDFFNSNREWRVYAY KDFKEEEKKLFLANIYDSNEFYKTIQIKEYDEQPTYSCQLLFKKDEEST DEIGLIGIHRFYESGIVLKDYKNYFCISKWYLKEVKRKPYNPNLGCNW QFIPKDEGWIE |
| SEQ ID NO: 10 | SLYNKTLDCRELLVKNTDLPFIGDISDVKTDIFLRKDINEETEVIYYPDN VSVDQVILSKNTSEHGQLDLLYPSIDSESEILPGENQVFYDNRTQNVDY LNSYYYLESQKLSDNVEDFTFTRSIEEALDNSAKVYTYFPTLANKVNA GVQGGLFLMWANDVVEDFTTNILRKDTLDKISDVSAIIPYIGPALNISN SVRRGNFTEAFAVTGVTILLEAFPEFTIPALGAFVIYSKVQERNEIIKTID NCLEQRIKRWKDSYEWMMGTWLSRIITQFNNISYQMYDSLNYQAGAI KAKIDLEYKKYSGSDKENIKSQVENLKNSLDVKISEAMNNINKFIREC SVTYLFKNMLPKVIDELNEFDRNTKAKLINLIDSHNIILVGEVDKLKAK VNNSFQNTIPFNIFSYTNNSLLKDIINEYFNNINDSKILSLQNRKNTLVD TSGYNAEVSEEGDVQLNPIFPFDFKLGSSGEDRGKVIVTQNENIVYNS MYESFSISFWIRINKWVSNLPGYTIIDSVKNNSGWSIGIISNFLVFTLKQ NEDSEQSINFSYDISNNAPGYNKWFFVTVTNNMMGNMKIYINGKLID TIKVKELTGINFSKTITFEINKIPDTGLITSDSDNINMWIRDFYIFAKELD GKDINILFNSLQYTNVVKDYWGNDLRYNKEYYMVNIDYLNRYMYAN SRQIVFNTRRNNNDFNEGYKIIIKRIRGNTNDTRVRGGDILYFDMTINN |
| | KAYNLFMKNETMYADNHSTEDIYAIGLREQTKDINDNIIFQIQPMNNT YYYASQIFKSNFNGENISGICSIGTYRFRLGGDWYRHNYLVPTVKQGN YASLLESTSTHWGFVPVSE |
| SEQ ID NO: 11 | DDSTCIKVKNNRLPYVADKDSISQEIFENKIITDETNVQNYSDKFSLDE SILDGQVPINPEIVDPLLPNVNMEPLNLPGEEIVFYDDITKYVDYLNSY YYLESQKLSNNVENITLTTSVEEALGYSNKIYTFLPSLAEKVNKGVQA GLFLNWANEVVEDFTTNIMKKDTLDKISDVSVIIPYIGPALNIGNSALR GNFNQAFATAGVAFLLEGFPEFTIPALGVFTFYSSIQEREKIIKTIENCLE QRVKRWKDSYQWMVSNWLSRITTQFNHINYQMYDSLSYQADAIKAK IDLEYKKYSGSDKENIKSQVENLKNSLDVKISEAMNNINKFIRECSVTY LFKNMLPKVIDELNKFDLRTKTELINLIDSHNIILVGEVDRLKAKVNES FENTMPFNIFSYTNNSLLKDIINEYFNSINDSKILSLQNKKNALVDTSGY NAEVRVGDNVQLNTIYTNDFKLSSSGDKIIVNLNNNILYSAIYENSSVS FWIKISKDLTNSHNEYTIINSIEQNSGWKLCIRNGNIEWILQDVNRKYK SLIFDYSESLSHTGYTNKWFFVTITNNIMGYMKLYINGELKQSQKIEDL DEVKLDKTIVFGIDENIDENQMLWIRDFNIFSKELSNEDINIVYEGQILR NVIKDYWGNPLKFDTEYYIINDNYIDRYIAPESNVLVLVQYPDRSKLYT GNPITIKSVSDKNPYSRILNGDNIILHMLYNSRKYMIIRDTDTIYATQGG ECSQNCVYALKLQSNLGNYGIGIFSIKNIVSKNKYCSQIFSSFRENTML LADIYKPWRFSFKNAYTPVAVTNYETKLLSTSSFWKFISRDPGWVE |
| SEQ ID NO: 12 | KSICIEINNGELFFVASENSYNDDNINTPKEIDDTVTSNNNYENDLDQVI LNFNSESAPGLSDEKLNLTIQNDAYIPKYDSNGTSDIEQHDVNELNVFF YLDAQKVPEGENNVNLTSSIDTALLEQPKIYTFFSSEFINNVNKPVQAA LFVGWIQQVLVDFTTEANQKSTVDKIADISIVVPYIGLALNIGNEAQK GNFKDALELLGAGILLEFEPELLIPTILVFTIKSFLGSSDNKNKVIKAINN ALKERDEKWKEVYSFIVSNWMTKINTQFNKRKEQMYQALQNQVNA LKAIIESKYNSYTLEEKNELTNKYDIEQIENELNQKVSIAMNNIDRFLTE SSISYLMKLINEVKINKLREYDENVKTYLLDYIIKHGSILGESQQELNS MVIDTLNNSIPFKLSSYTDDKILISYFNKFFKRIKSSSVLNMRYKNDKY VDTSGYDSNININGDVYKYPTNKNQFGIYNDKLSEVNISQNDYIIYDN KYKNFSISFWVRIPNYDNKIVNVNNEYTIINCMRDNNSGWKVSLNHN EIIWTLQDNSGINQKLAFNYGNANGISDYINKWIFVTITNDRLGDSKLY INGNLIDKKSILNLGNIHVSDNILFKIVNCSYTRYIGIRYFNIFDKELDET EIQTLYNNEPNANILKDFWGNYLLYDKEYYLLNVLKPNNFINRRTDST LSINNIRSTILLANRLYSGIKVKIQRVNNSSTNDNLVRKNDQVYINFVAS KTHLLPLYADTATTNKEKTIKISSSGNRFNQVVVMNSVGNCTMNFKN NNGNNIGLLGFKADTVVASTWYYTHMRDNTNSNGFFWNFISEEHGW QEK |
| SEQ ID NO: 13 | APPRLCIRVNNSELFFVASESSYNENDINTPKEIDDTTNLNNNYRNNLD EVILDYNSQTIPQISNRTLNTLVQDNSYVPRYDSNGTSEIEEYDVVDFN VFFYLHAQKVPEGETNISLTSSIDTALLEESKDIFFSSEFIDTINKPVNAA LFIDWISKVIRDFTTEATQKSTVDKIADISLIVPYVGLALNIIIEAEKGNF EEAFELLGVGILLEFVPELTIPVILVFTIKSYIDSYENKNKAIKAINNSLIE REAKWKEIYSWIVSNWLTRINTQFNKRKEQMYQALQNQVDAIKTAIE |
| | YKYNNYTSDEKNRLESEYNINNIEEELNKKVSLAMKNIERFMTESSIS YLMKLINEAKVGKLKKYDNHVKSDLLNYILDHRSILGEQTNELSDLV TSTLNSSIPFELSSYTNDKILIIYFNRLYKKIKDSSILDMRYENNKFIDISG YGSNISINGNVYIYSTNRNQFGIYNSRLSEVNIAQNNDIIYNSRYQNFSI SFWVRIPKHYKPMNHNREYTIINCMGNNNSGWKISLRTVRDCEIIWTL QDTSGNKENLIFRYEELNRISNYINKWIFVTITNNRLGNSRIYINGNLIV EKSISNLGDIHVSDNILFKIVGCDDETYVGIRYFKVFNTELDKTEIETLY SNEPDPSILKNYWGNYLLYNKKYYLFNLLRKDKYITLNSGILNINQQR GVTEGSVFLNYKLYEGVEVIIRKNGPIDISNTDNFVRKNDLAYINVVD RGVEYRLYADTKSEKEKIIRTSNLNDSLGQIIVMDSIGNNCTMNFQNN NGSNIGLLGFHSNNLVASSWYYNNIRRNTSSNGCFWSSISKENGWKE |
| SEQ ID NO: 14 | SEQCIIVNNEDLFFIANKDSFSKDLAKAETIAYNTQNNTIENNFSIDQLI LDNDLSSGIDLPNENTEPFTNFDDIDIPVYIKQSALKKIFVDGDSLFEYL HAQTFPSNIENLQLTNSLNDALRNNNKVYTFFSTNLVEKANTVVGASL FVNWVKGVIDDFTSESTQKSTIDKVSDVSIIIPYIGPALNVGNETAKENF KNAFEIGGAAILMEFIPELIVPIVGFFTLESYVGNKGHIIMTISNALKKR DQKWTDMYGLIVSQWLSTVNTQFYTIKERMYNALNNQSQAIEKIIED QYNRYSEEDKMNINIDFNDIDFKLNQSINLAINNIDDFINQCSISYLMN RMIPLAVKKLKDFDDNLKRDLLEYIDTNELYLLDEVNILKSKVNRHLK DSIPFDLSLYTKDTILIQVFNNYISNISSNAILSLSYRGGRLIDSSGYGAT MNVGSDVIFNDIGNGQFKLNNSENSNITAHQSKFVVYDSMFDNFSINF WVRTPKYNNNDIQTYLQNEYTIISCIKNDSGWKVSIKGNRIIWTLIDVN AKSKSIFFEYSIKDNISDYINKWFSITITNDRLGNANIYINGSLKKSEKIL NLDRINSSNDIDFKLINCTDTTKFVWIKDFNIFGRELNATEVSSLYWIQS STNTLKDFWGNPLRYDTQYYLFNQGMQNIYIKYFSKASMGETAPRTN FNNAAINYQNLYLGLRFIIKKASNSRNINNDNIVREGDYIYLNIDNISDE SYRVYVLVNSKEIQTQLFLAPINDDPTFYDVLQIKKYYEKTTYNCQILC EKDTKTFGLFGIGKFVKDYGYVWDTYDNYFCISQWYLRRISENINKL RLGCNWQFIPVDEGWTE |

Botulinum toxin-related products have broad application prospects in the treatment of clinical spastic diseases, hypersecretion of glands, neuropathic pain, and cosmetic wrinkle removal. However, as macromolecules, these products are currently administered by injection and are difficult to directly penetrate the skin to reach the site of action.

Currently, only type A and type B botulinum toxins can be used as drugs, but the vast majority used in the medical field is type A botulinum toxin. The type A botulinum toxins currently used clinically include Botox produced by Allergan in the United States, Dysport produced by Speywood in the United Kingdom, and BTX-A produced by Lanzhou Institute of Biological Products in China; botulinum toxin-related products have broad application prospects in the treatment of clinical spastic diseases, hypersecretion of glands, neuropathic pain, and cosmetic wrinkle removal. However, these products are currently administered by injection, whereas our macromolecular transdermal microemulsion system has been verified by pharmacodynamic experiments in mice to enable biological macromolecules such as botulinum toxin to achieve the intended effect through transdermal delivery.

Yeast extract (also known as yeast flavor, English name Yeast extract), abbreviated as YE, is a brown-yellow soluble paste or light yellow powder natural product prepared according to the Chinese Pharmacopoeia by using edible yeast rich in protein as raw material, employing modern biotechnology such as autolysis, enzymatic hydrolysis, separation, and concentration to degrade and refine proteins, nucleic acids and other components inside yeast cells. The main components of yeast extract, in addition to amino acids and nucleotides, also include monosaccharides, polysaccharides, minerals, vitamins, and others.

The main application efficacies of yeast extract are skin rejuvenation, anti-aging, and water-locking and moisturizing. Yeast extract has a very good skin rejuvenation effect, which is mainly achieved through three modes of action: (1) reducing the transfer of already produced melanin to the epidermal layer cells; (2) promoting the metabolism of melanin-containing corneocytes to accelerate stratum corneum shedding; (3) promoting the synthesis of epidermal layer proteins to regulate skin texture. Yeast extract has a very good free radical scavenging anti-aging effect, can regulate skin texture, fine lines and wrinkles, increase skin elasticity, make the skin smooth and delicate, and can also alleviate and prevent dull skin color problems occurring in the early aging process of the skin. Yeast extract also has a strong water-locking effect: yeast extract is rich in natural moisturizing factors, and can promote the stratum corneum to more effectively retain moisture, thus providing deep hydration to the skin, and preventing skin dryness; yeast extract can strengthen skin barrier function by increasing the content of ceramide in the stratum corneum, and reduce transepidermal water loss; yeast extract can also repair the damaged stratum corneum lipid barrier, improve skin resistance, and regulate skin condition.

Soluble collagen is a natural protein that has an important function in the skin. Collagen can maintain skin elasticity and firmness, but with aging, collagen in the skin gradually decreases, leading to skin sagging and the appearance of wrinkles. Soluble collagen, as a form that can penetrate the skin more deeply, can supplement collagen in the skin, thereby improving the condition of the skin.

Soluble collagen can stimulate and promote the synthesis of collagen, help repair damaged collagen, and promote the generation of new collagen, so that skin elasticity and firmness are improved, and wrinkles and fine lines are improved.

Elastin is an important component of skin composition, wherein elastin is the foundation of skin youth. Elastin peptides have effects such as promoting skin damage repair, vascular regeneration, and promoting fibroblast proliferation. Elastin can also enhance human skin elasticity, thereby making the skin smooth and elastic.

In one example, the composition of the macromolecular transdermal microemulsion system of the present invention is shown in Table 5:

| Table 5 | | |
|---|---|---|
| Name of composition | Type | By mass percentage based on the total weight of the transdermal composition |
| Glycerol | Oil phase | 5%-20% or 10%-20% or 13%-18% |
| Butylene glycol or propylene glycol | | 1%-10% or 3%-8% |
| Laurocapram | | 0.5%-5% or 1%-3% |
| Emulsifier (for example, any one or more of polysorbate 80, polysorbate 60, polysorbate 20, and sorbitan oleate) | Emulsifier | 5%-20% or 5-15% or 8-12% |
| Carboxymethyl deacetylated chitin (chitosan) or derivative of chitosan | Aqueous phase | 0.3%-3% or 1-2% |
| Water | | 55%-80% or 60%-70% |
| Macromolecular active substance | | Any described component type and effective amount of the foregoing |

Preferably, the example of the Table 5 is the composition of Table 6:

| Table 6 | | |
|---|---|---|
| Name of composition | Type | By mass percentage based on the total weight of the transdermal composition |
| Glycerol | Oil phase | 10%-20% or 13%-18% |
| Butylene glycol or propylene glycol | | 3%-8% |
| Laurocapram | | 1%-3% |
| Emulsifier (for example, any one or more of polysorbate 80, polysorbate 60, polysorbate 20, and sorbitan oleate) | Emulsifier | 5%-15% or 8%-12% |
| Carboxymethyl deacetylated chitin (chitosan) or derivative of chitosan | Aqueous phase | 1%-2% |
| Water | | 55%-80% or 60%-70% |
| Macromolecular active substance | | Water-soluble, and the content is less than or equal to 5%; or less than or equal to 2%; or less than or equal to 1%; or less than or equal to 0.05%; or less than or equal to 0.02%, preferably 0.00001%-0.015%, more preferably 0.0005%-0.015% or 0.0008%-0.0015%; |

Chitosan, as a natural molecular alkaline polysaccharide, has film-forming property, antibacterial property, and anti-inflammatory, hemostasis, pain relief, anti-pruritic, scar removal, promotion of wound healing, and antioxidant effects. Chitosan has good inhibitory effects on bacteria, yeast, fungi and other microorganisms, and has significant inhibitory effects on bacteria generally present on human epidermis such as *Staphylococcus epidermidis*, *Escherichia coli* and *Candida albicans*, as well as on *Pseudomonas aeruginosa*, *Staphylococcus aureus* and *pyogenic Staphylococcus aureus* infections easily occurring in burn patients. Chitosan has strong moisturizing property, with moisturizing ability even exceeding sodium hyaluronate, and is known as a "super moisturizer". Chitosan can absorb heavy metals and can absorb residues in cosmetics. Chitosan has repairing ability, can repair the skin barrier, and is useful for sensitive skin and acne-prone skin.

In one example, the macromolecular transdermal microemulsion system further comprises a preservative, preferably, by the mass percentage based on the total weight of the transdermal microemulsion system: 0.1%-1% of a preservative based on the mass percentage. Furthermore, the preservative is selected from any one or more of phenoxyethanol, methylparaben, ethylparaben, propylparaben, benzyl alcohol, and sorbic acid.

The particle size of the particles of the macromolecular transdermal microemulsion system of the present invention is on average nanoscale, preferably, 10-15 nm on average.

Table 5 is a specific example of the composition of the macromolecular transdermal microemulsion system provided by the present invention.

| Table 5 | | |
|---|---|---|
| Name of composition | Type | By mass percentage based on the total weight of the transdermal composition |
| Glycerol | Oil phase | 5%-20% or 10%-20% or 13%-18% |
| Butylene glycol or propylene glycol | | 1%-10% or 3%-8% |
| Laurocapram | | 0.5%-5% or 1%-3% |
| Emulsifier (for example, any one or more of polysorbate 80, polysorbate 60, polysorbate 20, and sorbitan oleate) | Emulsifier | 5%-20% or 5-15% or 8-12% |
| Carboxymethyl deacetylated chitin (chitosan) or derivative of chitosan | Aqueous phase | 0.3%-3% or 1-2% |
| Water | | 55%-80% or 60%-70% |
| Botulinum toxin type A | | 10 ng/mL-10 µg/mL |

The macromolecular transdermal microemulsion system of the present invention is applied in achieving therapeutic, cosmetic, and skin care effects, and during use, transdermal administration can be performed by any one or more of topical application, transdermal instrument, patch application, and spray.

The macromolecular transdermal microemulsion system of the present invention can also be applied in the preparation of drugs or products for achieving therapeutic, cosmetic, and skin care effects.

In addition, the water of the present invention is preferably any one of or a combination of more than one of distilled water, drinking water, sterile water, ultrapure water, and deionized water.

In the following embodiments, some component descriptions involved are as follows:

The application of superoxide dismutase in cosmetics mainly includes: (1) as a cosmetic additive, which can prevent skin aging and achieve skin care effects; (2) prevention and treatment of related skin diseases, which have been widely applied abroad. Currently, many high-end cosmetics, both domestically and internationally, have incorporated SOD and formulated into products such as facial masks, emulsions, and creams.

Vitamins are essential organic substances for the human body, vitamin A can promote the proliferation of epidermal cells, increase the formation of dermal collagen and elastin, and have anti-aging effects. Vitamin E is a natural antioxidant, which can promote metabolism and improve skin elasticity. Vitamin C has strong antioxidant properties, can promote collagen synthesis and inhibit collagen degradation, vitamin C and vitamin E have a synergistic effect in scavenging free radicals.

In addition to vitamins essential for the human body, various natural plant active ingredients have been proven to have anti-wrinkle and firming functions, such as Centella asiatica extract, Cladosiphon okamuranus extract, Calendula officinalis extract, etc. These raw materials have been widely used in anti-wrinkle and firming cosmetics.

### Example 1

The formulation of the macromolecular transdermal microemulsion system provided in this example is shown in Table 6:

| Table 6 | | |
|---|---|---|
| | Composition | Amount (by mass percentage) |
| 1 | Glycerol | 15% |
| 2 | Polysorbate-80 | 10% |
| 3 | Propylene glycol | 5% |
| 4 | Laurocapram | 2% |
| 5 | Carboxymethyl deacetylated chitin | 1.5% |
| 6 | Phenoxyethanol | 0.5% |
| 7 | Botulinum protein A | 0.01% |
| 8 | Purified water | Remainder |

Preparation method:
(1) Using an electronic balance, purified water, carboxymethyl deacetylated chitin, and botulinum protein A were sequentially weighed into a 3 L beaker 1 according to the formula amount, and stirred and dissolved at room temperature (rotation speed: 400-500 rpm).
(2) Using an electronic balance, polysorbate-80, laurocapram, phenoxyethanol, propylene glycol, and glycerol were sequentially weighed into a 2 L beaker 2 according to the formula amount, and stirred evenly at room temperature (rotation speed: 250-300 rpm).
(3) The solution in beaker 2 was added into beaker 1, and stirred evenly at room temperature (rotation speed: 400-500 rpm).
(4) Purified water was added to the solution prepared in step (3) to make up to 1000 g, and stirred and mixed evenly at room temperature.

### Example 2

The formulation of the macromolecular transdermal microemulsion system provided in this example is shown in Table 7.

| Table 7 | | |
|---|---|---|
| | Composition | Amount (by mass percentage) |
| 1 | Glycerol | 15% |
| 2 | Polysorbate-80 | 10% |
| 3 | Butylene glycol | 5% |
| 4 | Carboxymethyl deacetylated chitin | 1.5% |
| 5 | Yeast extract | 1% |
| 6 | Tocopherol (vitamin E) | 1% |
| 7 | Sodium ascorbyl phosphate | 1% |
| 8 | Superoxide dismutase (SOD) | 0.5% |
| 9 | Phenoxyethanol | 0.5% |
| 10 | Retinyl palmitate | 0.3% |
| 11 | (Daily use) fragrance | 0.2% |
| 12 | Purified water | Remainder |

Preparation method:
(1) Using an electronic balance, purified water, carboxymethyl deacetylated chitin, yeast extract, superoxide dismutase (SOD) and sodium ascorbyl phosphate were sequentially weighed into a 3 L beaker 1 according to the formula amount, and stirred and dissolved at room temperature (rotation speed: 400-500 rpm).
(2) Using an electronic balance, polysorbate-80, tocopherol (vitamin E), retinyl palmitate, phenoxyethanol, (daily) fragrance, butylene glycol and glycerol were sequentially weighed into a 2 L beaker 2 according to the formula amount, and stirred evenly at room temperature (rotation speed: 250-300 rpm).
(3) The solution in beaker 2 was added into beaker 1, and stirred evenly at room temperature (rotation speed: 400-500 rpm).
(4) Purified water was added to the solution prepared in step (3) to make up the volume, and stirred and mixed evenly at room temperature.

### Example 3

The formulation of the macromolecular transdermal microemulsion system provided in this example is shown in Table 8.

| Table 8 | | |
|---|---|---|
| Serial number | Composition | Amount (by mass percentage) |
| 1 | Glycerol | 15% |
| 2 | Polysorbate-80 | 10% |
| 3 | Butylene glycol | 5% |
| 4 | Carboxymethyl deacetylated chitin | 1.5% |
| 5 | Yeast extract | 1% |
| 6 | Tocopherol (vitamin E) | 1% |
| 7 | Sodium ascorbyl phosphate | 1% |
| 8 | Superoxide dismutase (SOD) | 0.5% |
| 9 | Phenoxyethanol | 0.5% |
| 10 | Retinyl palmitate | 0.3% |
| 11 | (Daily use) fragrance | 0.2% |
| 12 | Soluble collagen | 0.01% |
| 13 | Elastin | 0.01% |
| 14 | Hydroxypropyl tetrahydropyrantriol | 0.01% |
| 15 | Carnosine | 0.01% |
| 16 | Sodium polyglutamate | 0.01% |
| 17 | Cladosiphon okamuranus extract | 0.02% |
| 18 | Calendula officinalis extract | 0.02% |
| 19 | Centella asiatica extract | 0.02% |
| 20 | Aloe extract | 0.02% |
| 21 | Biotin | 0.01% |
| 22 | Purified water | Remainder |

Preparation method:
(1) Using an electronic balance, purified water, hydroxypropyl tetrahydropyrantriol, carnosine, sodium polyglutamate, biotin, cladosiphon okamuranus extract, Calendula officinalis extract, Centella asiatica extract, aloe extract, carboxymethyl deacetylated chitin, sodium ascorbyl phosphate, yeast extract, superoxide dismutase (SOD), soluble collagen, and elastin were sequentially weighed into a 3 L beaker 1, stirred and dissolved at room temperature (rotation speed: 400-500 rpm).
(2) Using an electronic balance, polysorbate-80, tocopherol (vitamin E), retinyl palmitate, phenoxyethanol, (daily) fragrance, butylene glycol and glycerol were sequentially weighed into a 2 L beaker 2 according to the formula amount, and stirred evenly at room temperature (rotation speed: 250-300 rpm).
(3) The solution in beaker 2 was added into beaker 1, and stirred evenly at room temperature (rotation speed: 400-500 rpm).
(4) Purified water was added to the solution prepared in step (3) to make up to 1000 g, and stirred and mixed evenly at room temperature.

The formulations obtained in examples 1-3 have good emulsification effect, high transparency, and no stratification; the average particle size detected by a nanoparticle size potentiometer is 10-15 nm. In particular, the average particle size of the formulation obtained in example 3 is 12.99 nm.

### Comparative example 1

The formulation of this comparative example is shown in Table 9:

| Table 9 | |
|---|---|
| **Composition** | **Amount (by mass percentage)** |
| Oleic acid | 10.283% |
| Oleyl alcohol | 12.754% |
| Propylene glycol | 11.592% |
| Menthol | 1.000% |
| Laurocapram | 2.000% |
| Polysorbate 80 | 35.574% |
| Sorbitan oleate | 13.118% |
| Phenoxyethanol | 0.500% |
| Botulinum protein type A | 0.001% |
| Purified water | 13.178% |

Preparation method:
(1) The acid ester was added into a 2 L beaker and stirred for 5 min; oleyl alcohol and oleic acid were weighed according to the formula amount using an electronic balance into the beaker and stirred for 5 min; laurocapram and phenoxyethanol were weighed according to the formula amount using an electronic balance into the beaker and stirred evenly at room temperature (rotation speed: 200-250 rpm, 5 min).
(2) Propylene glycol and menthol were weighed according to the formulation using an electronic balance and were added successively into a blue cap bottle, fully dissolved by heating to 60°C (about 10 min), and then added to the solution obtained in step (1), and stirred evenly at room temperature (rotation speed: 200-250 rpm, 5 min).
(3) 10 mg of botulinum protein A was added to the solution prepared in (2), and stirred evenly at room temperature (rotation speed: 200-250 rpm, 5 min).
(4) Purified water was added to the solution prepared in step (3) to make up to 1000 g, and stirred and mixed evenly at room temperature.

### Comparative examples 2-10

The formulations of comparative examples 2-10 are shown in Table 10, based on mass percentage:

| Table 10 | | | | | | | | | |
|---|---|---|---|---|---|---|---|---|---|
| Name | Compar ative example 2 | Compar ative example 3 | Compar ative example 4 | Compar ative example 5 | Compar ative example 6 | Compar ative example 7 | Compar ative example 8 | Compar ative example 9 | Comparati ve example 10 |
| Glycerol | 20.000% | 15.000% | 15.000% | 15.000% | 15.000% | 15.000% | 15.000% | 15.000% | 15.000% |
| Polysorbate 80 | 2.000% | 5.000% | 5.000% | 5.000% | 5.000% | 5.000% | 10.000 % | 10.000 % | 10.000% |
| Polysorbate-20 | 0.000% | 0.000% | 0.000% | 0.000% | 0.000% | 0.000% | 0.000% | 5.000% | 10.000% |
| Sorbitan oleate | 0.000% | 0.000% | 0.000% | 0.000% | 0.000% | 0.000% | 3.750% | 0.000% | 0.000% |
| Propylene glycol | 2.000% | 5.000% | 5.000% | 5.000% | 5.000% | 5.000% | 5.000% | 5.000% | 5.000% |
| Laurocapram | 2.000% | 0.000% | 0.000% | 0.000% | 0.000% | 0.000% | 0.000% | 0.000% | 0.000% |
| Phenoxyethan ol | 0.500% | 0.500% | 0.500% | 0.500% | 0.500% | 0.500% | 0.500% | 0.500% | 0.500% |
| Carboxymeth yl deacetylated chitin (chitosan) | 1.000% | 1.000% | 1.500% | 1.500% | 1.500% | 1.500% | 1.500% | 1.500% | 1.500% |
| Yeast extract | 1.000% | 1.000% | 1.000% | 1.000% | 1.000% | 1.000% | 1.000% | 1.000% | 1.000% |
| Tocopherol (vitamin E) | 0.000% | 0.000% | 1.000% | 1.000% | 0.000% | 0.300% | 0.000% | 0.550% | 0.450% |
| Sodium ascorbyl phosphate | 0.000% | 0.000% | 0.500% | 0.500% | 0.500% | 0.500% | 0.500% | 0.500% | 0.500% |
| Retinyl palmitate | 0.000% | 0.000% | 0.900% | 0.000% | 0.500% | 0.300% | 0.000% | 0.550% | 0.400% |
| (Daily use) fragrance | 0.100% | 0.050% | 0.200% | 0.200% | 0.200% | 0.200% | 0.200% | 0.200% | 0.200% |
| Soluble collagen | 0.000% | 0.000% | 0.000% | 0.000% | 0.000% | 0.000% | 0.000% | 0.000% | 0.000% |
| Hamamelis mollis extract | 0.000% | 0.000% | 0.050% | 0.050% | 0.050% | 0.050% | 0.050% | 0.050% | 0.050% |
| Centella asiatica extract | 0.000% | 0.000% | 0.050% | 0.050% | 0.050% | 0.050% | 0.050% | 0.050% | 0.050% |
| Water | 71.400 % | 72.450 % | 69.300% | 70.200 % | 70.700 % | 70.600 % | 62.450 % | 60.100 % | 55.350% |

According to the formulations of comparative examples 2-10, the oil phases were fully mixed and dissolved respectively, the aqueous phases were fully mixed and dissolved respectively, and then the phases were mixed to obtain the product. The specific preparation method was a conventional technology and can be selected with reference to the previous examples, so that it will not be described one by one.

The comparative examples 2-10 obtained by preparation had worse appearance effects compared with examples 1-3, specifically as shown in Table 11:

| Table 11 | |
|---|---|
| Comparative example | Appearance effect |
| Comparative example 2 | Emulsion effect was poor, transparency was poor, and layering was observed |
| Comparative example 3 | Emulsion effect was poor, transparency was poor, and layering was observed |
| Comparative example 4 | Emulsion effect was poor, transparency was poor, and layering was observed |
| Comparative example 5 | Emulsion effect was poor, transparency was poor, and layering was observed |
| Comparative example 6 | Emulsion effect was poor, transparency was poor, and layering was observed |
| Comparative example 7 | Emulsion effect was poor, transparency was poor, and layering was observed |
| Comparative example 8 | Emulsion effect was poor, transparency was poor, and layering was observed |
| Comparative example 9 | The stickiness was too strong |
| Comparative example 10 | The stickiness was too strong |

### Example 4

In this experiment, the transdermal permeation efficiency was determined.

The botulinum protein A added in example 1 and comparative example 1 was replaced with BSA protein, wherein the BSA protein emulsion prepared in example 1 was 1.0 mg/mL, and the BSA protein emulsion prepared in comparative example 1 was 0.62 mg/mL, the transdermal experiment was conducted by the Franz diffusion cell method.

### Experimental equipment and materials

1. Reagent
   Phosphate buffer solution, protein standard, test article, protein detection kit (ultrahigh sensitivity);
2. Consumables
   Ex vivo porcine skin for transdermal evaluation, centrifuge tubes, EP tubes, pipette tips, pre-coated microplate;
3. Equipment
   TP-6 transdermal diffusion instrument (Tianjin Jintuo Instrument Technology Co., Ltd.), multi-functional microplate reader (Thermo Scientific, Varioskan Lux), medical refrigerator (Qingdao Haier Biomedical Co., Ltd.), biochemical incubator (Shanghai Yiheng Scientific Instrument Co., Ltd.), pipettor (Eppendorf AG).

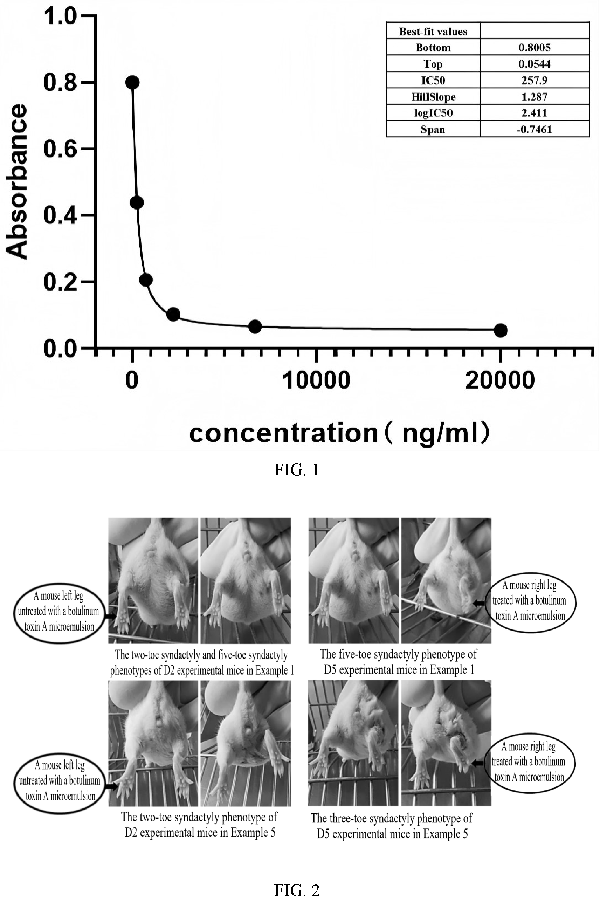

I. Preparation before the experiment:
   (1) The porcine skin required for the experiment was taken out from the -20°C refrigerator and thawed at 4°C, then washed with physiological saline and set aside for later use.
   (2) Before the experiment, the donor chamber, diffusion chamber (receptor chamber) receiving liquid from the donor chamber, and the stirring bar were cleaned.
   (3) Purified water was added to the water tank, level with the diffusion cell rack, the temperature was set to 32°C, and the rotation speed was set.
   (4) After the temperature of the TP-6 transdermal diffusion instrument rose to the set temperature, the porcine skin was placed between the donor chamber and the diffusion chamber. The solution was stirred at a constant speed to maintain it at an isothermal state and uniform concentration of the permeate.
II. Experimental method:
   (1) 3 experimental groups and one blank group were set.
   (2) BSA protein test solution was added: 150 µLof the BSA protein emulsion test solution (1 mg/mL) prepared in example 1, 150 µL of the BSA protein emulsion test solution prepared in comparative example 1 (0.62 mg/mL), 150 µL of the BSA protein test solution dissolved in PBS (1 mg/mL) were added to the first 3 donor chambers, respectively, and 150 µL of PBS was added to the blank group.
   (3) The receptor chamber was filled with PBS. Four 1.5 mL EP tubes were taken, and 400 ul of liquid was taken from the sampling port of the receptor chamber every 1 hour for subsequent OD detection. The sampling time and experimental group number were marked, with a total sampling time of 6 hours. After sampling, each receptor chamber must be replenished with 400 µL of PBS to maintain the volume of the receptor chamber unchanged.
   (4) All collected samples were placed in a 4°C refrigerator and set aside for later use.
III. ELISA experimental detection:
   1. Preparation of reagents:
      a. all reagents were equilibrated to room temperature (18-25°C) before use. The detection wavelength was set to 450 nm according to the microplate reader manual and the instrument was preheated for 15 minutes before reading the plate.
      b. Washing buffer: 20 mL of concentrated washing buffer was diluted with 580 mL of deionized water to prepare 600 mL of washing buffer.
      c. Standard working solution: First, the 1000 g of standard was centrifuged for 1 min, then 2 mL of sample diluent for the standard was added, and the standard was mixed with the sample diluent for the standard. 300 µL of 20000 ng/mL standard diluent was taken into the first tube and mixed to obtain a 6666.7 ng/mL working solution. Then 300 µL of solution was transferred from the previous tube to the next tube. Before the next transfer, each tube was thoroughly mixed. 6 dilution points for the standard were set, which were 20000 ng/mL, 6666.7 ng/mL, 2222.2 ng/mL, 740.7 ng/mL, 246.9 ng/mL, and 0 ng/mL, respectively.
      d. Detection reagent A working solution: the required amount was calculated before the experiment (100 µL/well). During the preparation, it was advisable to prepare 100-200 µL more than the calculated volume. Before use, the original tube was slightly centrifuged, and the 100× concentrated detection reagent A was diluted with detection diluent A to a 1× working solution A (e.g., 10 µL detection reagent A + 990 µL detection reagent A diluent).
      e. Detection reagent B working solution: the required amount was calculated before the experiment (100 µL/well). During the preparation, it was advisable to prepare 100-200 µLL more than the calculated volume. Before use, the original tube was slightly centrifuged, and the 100× concentrated detection reagent B was diluted with detection diluent B to a 1× working solution B (e.g., 10 µL detection reagent B + 990 µL detection reagent B diluent).
   2. Experimental method
      (1) Wells for standards, samples, and blank were set, respectively. 6 different concentrations of standards (including zero well, 50 µL/well) were added in sequence, 50 µL of the sample to be tested (sample collected from the sampling port of the receptor chamber) was added to other wells, then detection reagent A working solution was immediately added at 50 µL/well, and gently shaken to ensure thorough mixing, the wells were covered with the sealing film provided by the kit, and incubated at 37°C for 60 minutes.
      (2) The liquid in all wells was discarded, 350 uL of washing buffer was added to each well, soaked for 60 seconds, then the liquid in each well was poured out and the wells were blotted dry on clean absorbent paper. This washing step was repeated 3 times in total.
      (3) 100 µL of detection reagent B working solution was added to each well, the wells were covered with the sealing film, and incubated at 37°C for 30 minutes.
      (4) The liquid in each well was discarded, and the washing process of step 2 was repeated 5 times.
      (5) 90 µL of TMB reagent was added to each well, the wells were covered with a new sealing film, and incubated at 37°C in the dark for 10-20 minutes.
      (6) 50 µL of stop solution was added to each well in the same order as the addition of the chromogenic solution, and gently shaken to ensure thorough mixing.
      (7) It was ensured that there were no bubbles or water mist at the bottom of the wells of the microplate, and the absorbance OD value of each well was immediately measured at 450 nm, and the readings from the microplate reader was recorded.
   IV. Detection data and conclusions
      (1) The standard curve for the BSA standards obtained by four-parameter fitting is shown in FIG. 1.

### Standard curve plotting:

Processing of the detected standard protein OD value data:

The data were subjected to four-parameter fitting to obtain the standard curve, and the four-parameter fitting formula is: $y = bottom + \frac{top - bottom}{1 + {\left(\frac{IC 50}{x}\right)}^{HillSlope}}$

The specific standard curve is shown in FIG. 1.

### (2) Sample detection results

Table 12 shows the concentration of BSA permeated through the skin at different time points for different samples calculated according to the standard curve.

| Table 12 | | | |
|---|---|---|---|
| Time (h) | Example 1 Preparation of BSA emulsion (ng/mL) | Comparative example 1 Preparation of BSA emulsion (ng/mL) | BSA dissolved in PBS (ng/mL) |
| 0 | 0 | 0 | 0 |
| 1.5 | 105.8 | 95.2 | 68.0 |
| 3 | 151.8 | 147.1 | 108.7 |
| 6 | 148.6 | 129.1 | 110.0 |
| 12 | 125.8 | 142.7 | 125.4 |
| 24 | 239.6 | 134.1 | 114.7 |

According to the results, transdermal efficiency calculation was performed:
(1) For the BSA emulsion protein test solution prepared in example 1, the concentration in the receptor chamber after 24 hours was 239.6 ng/mL, with a volume of 15 mL. The concentration of BSA in the donor chamber was 1 mg/mL, and the volume added to the donor chamber was 150 µL. Based on these values, the transdermal penetration efficiency of the BSA emulsion prepared in example 1 was calculated to be 2.40%.
(2) For the BSA emulsion protein test solution prepared in comparative example 2, the concentration in the receptor chamber after 24 hours was 147.1 ng/mL, with a volume of 15 mL. The concentration of BSA in the donor chamber was 0.62 mg/mL, and the volume added to the donor chamber was 150 µL. Based on these values, the transdermal penetration efficiency of the BSA emulsion prepared in example 5 was calculated to be 2.37%.
(3) For the BSA protein test solution dissolved in PBS, the concentration in the receptor chamber after 24 hours was 125.4 ng/mL, with a volume of 15 mL. The concentration of BSA in the donor chamber was 1 mg/mL, and the volume added was 150 µL. Based on these data, the transdermal penetration efficiency for the PBS was calculated to be 1.25%.

It can be seen that both example 1 and comparative example 2 have very good transdermal effects relative to the control, and the transdermal effect of example 1 is better than that of comparative example 2.

### Example 5

Pharmacodynamic validation experiment of botulinum protein A microemulsion with a concentration of 10 µg/mL prepared in example 1 and comparative example 1 in mice.

In order to compare the difference in transdermal permeation efficiency of macromolecular active substance between example 1 and comparative example 1, SPF grade CD-1(ICR) mice weighing 17-19 g were used. After anesthesia with tribromoethanol, the skin on the right hind leg was shaved. Before each topical application, the application site was cleaned with medical cotton swabs dipped in 10% alcohol and dried, and then the drug was applied. 200 µL of botulinum protein A microemulsion was topically applied to the skin on the right hind leg of the mice once daily for 7 consecutive days. The left hind leg of the mice was not topically applied with botulinum protein A.

The experimental results showed that after 2-3 days of topical application of botulinum protein A, the toes of the right hind leg of the mice showed a toe syndactyly phenotype (syndactyly of toes 2-5); the experimental results of example 1 and comparative example 1 are shown in Table 13; the emulsion prepared with the formulation from example 1 induced this toe syndactyly phenotype as early as D2: 33% of the experimental mice exhibited syndactyly of two toes, while another 33% showed syndactyly of all five toes; Toe syndactyly status on D3: 33% of the experimental mice exhibited syndactyly of two toes, while another 33% showed syndactyly of all five toes; Toe syndactyly status on D4: 66% of the experimental mice exhibited syndactyly of three toes, while another 33% showed syndactyly of five toes; Toe syndactyly status on D5: 100% of the experimental mice exhibited syndactyly of five toes.

The emulsion prepared with the formulation of comparative example 1 exhibited no toe syndactyly on D2; Toe syndactyly status on D3: 33% of the experimental mice exhibited syndactyly of two toes; Toe syndactyly status on D4: 66% of the experimental mice exhibited syndactyly of two toes, while another 33% showed syndactyly of three toes; Toe syndactyly status on D5: 33% of the experimental mice exhibited syndactyly of three toes, while another 66% showed syndactyly of four toes.

| Table 13 | | | |
|---|---|---|---|
| Pharmacodynamic experimental results of botulinum protein A microemulsion with a concentration of 10 µg/mL prepared in example 1 and comparative example 1 in mice. | | | |
| | Composition | Experimental group of example 1 | Experimental group of comparative example 1 |
| 1 | Toe syndactyly status on D0 | 100% no toe syndactyly | 100% no toe syndactyly |
| 2 | Toe syndactyly status on D1 | 100% no toe syndactyly | 100% no toe syndactyly |
| 3 | Toe syndactyly status on D2 | 33% exhibited syndactyly of two toes, 33% exhibited syndactyly of five toes | 100% no toe syndactyly |
| 4 | Toe syndactyly status on D3 | 33% exhibited syndactyly of two toes, 33% exhibited syndactyly of five toes | 33% exhibited syndactyly of two toes |
| 5 | Toe syndactyly status on D4 | 66% exhibited syndactyly of three toes, 33% exhibited syndactyly of five toes | 66% exhibited syndactyly of two toes, 33% exhibited syndactyly of three toes |
| 6 | Toe syndactyly status on D5 | 100% exhibited syndactyly of five toes | 33% exhibited syndactyly of three toes, 66% exhibited syndactyly of four toes |

The results in Table 13 show that the pharmacodynamic experimental results of the botulinum protein A microemulsion prepared in example 1 in mice were significantly superior to those of comparative example 1, wherein the microemulsion of example 1 not only acted earlier but also had better pharmacodynamics than the microemulsion of comparative example 1. The toe syndactyly phenotype of example 1 and comparative example 1 is shown in FIG. 2.

According to literature reports, toe syndactyly phenotype appears after gastrocnemius muscle injection of botulinum protein A in mice.

It can be seen that by using the developed emulsion delivery system to prepare botulinum protein A into a microemulsion and topically applying it on mouse skin, the toe syndactyly phenotype after botulinum protein A injection was also achieved, thereby validating the developed emulsion system in vivo in mice with better effect.

### Example 7

In vivo efficacy detection in humans of the anti-wrinkle and firming cosmetic microemulsion prepared in example 3.
1. Under normal circumstances, adult subjects continuously used the product according to instructions for 28 days to evaluate whether the product had moisturizing, repairing, firming, anti-wrinkle effects and whether the product was suitable for sensitive skin and is mild and non-irritating.
2. Subjects: A total of 31 subjects completed the evaluation effectively, who were Chinese healthy females with sensitive skin (screened by SGS (SGS-CSTC Standards Technical Services Co., Ltd.) internal sensitive skin questionnaire), aged 31 to 60 years, with an average age of 52.65 ± 6.08 years, meeting the voluntary inclusion and exclusion criteria for subjects. The evaluation area was the face.

### Subject inclusion and exclusion criteria:

The subjects for this evaluation were selected from the SGS CPCH efficacy laboratory subject information database, wherein healthy subjects who met the following inclusion criteria and did not meet the following exclusion criteria were chosen.

### (1) Inclusion criteria

Healthy females, age: 28-60 years;
Race: Asian (Chinese);
subjects' facial skin is sensitive skin (screened by SGS internal sensitive skin questionnaire);
subjects self-report facial (non-persistent) itching or stinging discomfort symptoms scored 4-7 (0-9 scale, self-assessment questionnaire);
subjects self-report facial skin dullness, sagging, and lack of elasticity;
forehead wrinkle visual score 3-6 (according to SGS internal atlas);
random one side outer eye corner wrinkle visual score 3-6 (according to SGS internal atlas);
one side nasolabial fold wrinkle (smile lines) visual score 3-6 (according to SGS internal atlas);
transepidermal water loss (TEWL) on one side of the cheek area > 15 g/h/m²;
the measured value of stratum corneum water content on one side of the cheek area < 60 a.u.;
facial skin without obvious skin lesions, scars, hair, etc.;
able to cooperate well with the evaluation items according to the protocol requirements and maintain regular lifestyle during the study period.

### (2) Exclusion criteria

subjects with any of the following conditions must be excluded from this study:
those who do not agree to sign the informed consent form;
those unwilling to comply with the protocol requirements;
those simultaneously participating in any other clinical studies;
those using cosmetics and/or drugs on the day of evaluation;
those self-reporting to be pregnant or lactating;
those undergoing drug treatment during the study period;
subjects suffering from infectious skin diseases or atopic dermatitis;
those having skin abnormalities such as moles or telangiectasia at the evaluation site;
subjects who have undergone skin peeling or skin treatment within 3 months prior to participation in the evaluation;
subjects who have undergone immunosuppressive treatment within 3 months prior to participation in the evaluation;
subjects who have undergone systemic steroid treatment or phototherapy within 1 month prior to participation in the evaluation;
those who have used topical drugs and/or special effect (those claiming moisturizing, repairing, firming, anti-wrinkle) cosmetics on the involved site within 2 weeks prior to participation in the evaluation;
those having lesions, obvious marks or other abnormalities in the evaluation area, making measurement difficult;
subjects with severe reactions or allergies to cosmetics, drugs or general light exposure;

Besides the above matters, subjects who, in the judgment of the principal investigator, are otherwise unsuitable for the evaluation.

3. Method of use: After the skin was cleansed, oil was thoroughly removed and the skin was kept dry. An appropriate amount of essence (microemulsion) was taken and two drops were dropped with a dropper to the skin at the corners of the eyes, forehead, smile lines, lower jaw and other wrinkled areas, and the application sites were massaged until the essence (microemulsion) was absorbed. It was recommended to use every day for 7 days a week. A 4-week period was recommended as one usage cycle (during non-use periods, please store the product in a refrigerator at a constant temperature of 2-8°C).

4. Evaluation period: Before using the product (D0), after 14 days of using the product (D14), after 28 days of using the product (D28).

### 5. Evaluation parameters:

(1) Image acquisition
   Primos CR was used for facial image acquisition of skin wrinkles and photo analysis. The decrease in the analysis value indicated an improvement in skin wrinkles.
(2) Skin elasticity
   The skin elasticity measuring instrument Cutometer^{®} MPA580 was used to detect skin elasticity. An increase in the measurement values of R2, R5, and R7 indicated an improvement in skin elasticity.
(3) Skin firmness
   The skin elasticity measuring instrument Cutometer^{®} MPA580 was used to detect skin firmness. The decrease of the F4 measurement value indicated that the skin firmness was improved.
(4) Skin stratum corneum water content
   The skin water content meter Corneometer^{®} CM825 was used to detect the stratum corneum water content. The increase in the measurement value indicated that the stratum corneum water content was increased.
(5) Skin transepidermal water loss rate
   The skin water loss meter Tewameter^{®} TM Hex was used to detect the transepidermal water loss rate. The decrease in the measurement value indicated that the skin barrier was improved.
(6) TC value
   The TC value is a secondary parameter in non-invasive detection, representing the transepidermal water loss per unit area over a unit of time; the decrease of the analysis value indicated that the skin barrier function was improved.
(7) Skin moisture distribution map
   The skin moisture distribution meter MoistureMap MM 200 is a unique instrument based on capacitive imaging for observing moisture distribution and texture characteristics. MGL represents the mean gray value of the skin moisture distribution; the smaller the value, the higher the moisture.
(8) Skin glossiness
   The skin glossiness meter Glossymeter^{®} GL200 was used to detect the skin glossiness. The increase in the measurement value indicated that the skin glossiness was increased.
(9) Subject self-assessment
   The subject performs self-assessment based on their own usage.

### 6. Assessment protocol design:

### (1) Before product use (D0):

The subject arrived at SGS, cleansed the face with a cleansing product, and dried the skin with a lint-free dry facial tissue, then sat quietly for 30 min in a laboratory at 21 ± 1°C and 50 ± 10% RH, followed by visual assessment by a dermatologist; those meeting the inclusion criteria proceeded to the next assessment.

The laboratory technician measures the stratum corneum water content (Corneometer) and transepidermal water loss (Tewameter) of the facial skin; those meeting the inclusion criteria proceed to the next assessment.

The laboratory technician collects Primos CR images, Moisture Map MM200, Cutometer, and Glossymeter measurements from qualified subjects.

### (2) Subject fills out questionnaire

The laboratory technician explains the product usage method to the subject, distributes the product, and the subject tries the sample on-site under the supervision of the laboratory technician, wherein if adverse reactions occur, the usage log must be filled out promptly.

### (3) After 14 days of product use (D14)

The subject arrived at SGS, cleansed the face with a cleansing product, and dried the skin with a lint-free dry facial tissue, then sat quietly for 30 min in a laboratory at 21 ± 1°C and 50 ± 5% RH, followed by collection of the subject's facial Primos CR images and facial skin instrument measurements including Cutometer, Moisture Map MM200, Tewameter, Corneometer, and Glossymeter by the laboratory technician.

The laboratory technician weighed the product and checked the product usage log.

The subject left SGS.

### (4) After 28 days of product use (D28)

The subject arrived at SGS, cleansed the face with a cleansing product, and dried the skin with a lint-free dry facial tissue, then sat quietly for 30 min in a laboratory at 21 ± 1°C and 50 ± 5% RH, followed by collection of the subject's facial Primos CR images and facial skin instrument measurements including Cutometer, Moisture Map MM200, Tewameter, Corneometer, and Glossymeter by the laboratory technician.

The subject filled out the usage questionnaire.

The laboratory technician weighed and recovered the product, and inspected and recovered the product usage log.

The subject left SGS.

(5) Data statistics: The data were statistically analyzed using SPSS 28.0, and the evaluation data were tested for normal distribution; if the evaluation data were normally distributed, the T-test method was used for statistical analysis. If the evaluation data were not normally distributed, the rank sum test method was used for statistical analysis. Grade data were statistically analyzed using the rank sum test. The significance level of the statistical method was P < 0.05.

Through continuous use of the product for 28 days by 31 Chinese healthy female subjects with sensitive skin, the evaluation results showed that, under the evaluation conditions, the product already had moisturizing, repairing, firming, and anti-wrinkle effects at 14 days and was suitable for sensitive skin, mild, and non-irritating. The specific results are as follows

### (1) Instrument evaluation results

**(Table 14)**

| Table 14 In vivo efficacy detection in humans of the microemulsion prepared in example 3 | | | | | |
|---|---|---|---|---|---|
| Evaluation parameters (Evaluation instrument) (Evaluation site) | Time point | Rate of change | Significant result compared with before using the sample | Number of improved subjects (proportion of improved subjects) | Parameter description |
| Average skin wrinkle depth (Primos CR) (Forehead) | D14 | -10.25% | Significant difference | 19 (61%) | The decrease in the analysis value indicated an improvement in skin wrinkles |
| | D28 | -13.83% | Significant difference | 23 (74%) | |
| Skin wrinkle count (Primos CR) (Forehead) | D14 | -11.96% | Significant difference | 10 (32%) | The decrease in the analysis value indicated an improvement in skin wrinkles |
| | D28 | -19.57% | Significant difference | 15 (48%) | |
| Skin wrinkle volume (Primos CR) (Forehead) | D14 | -18.47% | Significant difference | 26 (84%) | The decrease in the analysis value indicated an improvement in skin wrinkles |
| | D28 | -36.34% | Significant difference | 31 (100%) | |
| Skin wrinkle area (Primos CR) (Forehead) | D14 | -9.47% | Significant difference | 22 (71%) | The decrease in the analysis value indicated an improvement in skin wrinkles |
| | D28 | -24.57% | Significant difference | 29 (94%) | |
| Skin wrinkle length (Primos CR) (Forehead) | D14 | -12.39% | Significant difference | 19 (61%) | The decrease in the analysis value indicated an improvement in skin wrinkles |
| | D28 | -23.41% | Significant difference | 29 (94%) | |
| Average skin wrinkle depth (Primos CR) (outer eye corner) | D14 | -8.46% | Significant difference | 23 (74%) | The decrease in the analysis value indicated an improvement in skin wrinkles |
| | D28 | -10.87% | Significant difference | 22 (71%) | |
| Skin wrinkle count (Primos CR) (outer eye corner) | D14 | -10.87% | Significant difference | 9 (29%) | The decrease in the analysis value indicated an improvement in skin wrinkles |
| | D28 | -21.74% | Significant difference | 16 (52%) | |
| Skin wrinkle volume (Primos CR) (outer eye corner) | D14 | -15.23% | Significant difference | 25 (81%) | The decrease in the analysis value indicated an improvement in skin wrinkles |
| | D28 | -26.16% | Significant difference | 31 (100%) | |
| Skin wrinkle area (Primos CR) (outer eye corner) | D14 | -5.43% | No significant difference | 23 (74%) | The decrease in the analysis value indicated an improvement in skin wrinkles |
| | D28 | -19.02% | Significant difference | 30 (97%) | |
| Skin wrinkle length (Primos CR) (outer eye corner) | D14 | -4.50% | No significant difference | 21 (68%) | The decrease in the analysis value indicated an improvement in skin wrinkles |
| | D28 | -23.60% | Significant difference | 27 (87%) | |
| Average skin wrinkle depth (Primos CR) (nasolabial fold) | D14 | -4.59% | Significant difference | 24 (77%) | The decrease in the analysis value indicated an improvement in skin wrinkles |
| | D28 | -9.25% | Significant difference | 25 (81%) | |
| Skin wrinkle volume (Primos CR) (nasolabial fold) | D14 | -5.46% | No significant difference | 18 (58%) | The decrease in the analysis value indicated an improvement in skin wrinkles |
| | D28 | -18.51% | Significant difference | 28 (90%) | |
| Skin wrinkle area (Primos CR) | D14 | -1.41% | No significant difference | 17 (55%) | The decrease in the analysis value |
| (nasolabial fold) | D28 | -13.45% | Significant difference | 26 (84%) | indicated an improvement in skin wrinkles |
| Skin wrinkle length (Primos CR) (nasolabial fold) | D14 | -1.57% | No significant difference | 15 (48%) | The decrease in the analysis value indicated an improvement in skin wrinkles |
| | D28 | -12.30% | Significant difference | 22 (71%) | |
| Skin elasticity R2 value (Cutometer^{®} dual MPA580) (cheek) | D14 | 6.55% | Significant difference | 26 (84%) | The increase in the measurement value indicated an improvement in skin elasticity |
| | D28 | 13.71% | Significant difference | 31 (100%) | |
| Skin elasticity R5 value (Cutometer^{®} dual MPA580) (cheek) | D14 | 12.84% | Significant difference | 26 (84%) | The increase in the measurement value indicated an improvement in skin elasticity |
| | D28 | 18.40% | Significant difference | 29 (94%) | |
| Skin elasticity R7 value (Cutometer^{®} dual MPA580) (cheek) | D14 | 16.24% | Significant difference | 29 (94%) | The increase in the measurement value indicated an improvement in skin elasticity |
| | D28 | 22.92% | Significant difference | 29 (94%) | |
| Skin firmness F4 value (Cutometer^{®} dual MPA580) (cheek) | D14 | -17.42% | Significant difference | 31 (100%) | The decrease in the measurement value indicated that the skin firmness was improved |
| | D28 | -29.53% | Significant difference | 31 (100%) | |
| Skin transepidermal water loss rate (Tewameter^{®} TM Hex) (cheek) | D14 | -5.62% | Significant difference | 30 (97%) | The decrease in the measurement value indicated that the skin barrier was improved |
| | D28 | -15.53% | Significant difference | 31 (100%) | |
| Skin stratum corneum water content (Corneometer^{®} CM825) (cheek) | D14 | 20.76% | Significant difference | 31 (100%) | The increase in the measurement value indicated that the stratum corneum water content was increased |
| | D28 | 47.99% | Significant difference | 31 (100%) | |
| TC value | D14 | -23.09% | Significant difference | 31 (100%) | The decrease in the measurement value indicated that the skin barrier was improved |
| | D28 | -43.83% | Significant difference | 31 (100%) | |
| Mean gray level MGL (MoistureMap MM 200) (cheek) | D14 | -9.11% | Significant difference | 29 (94%) | MGL represents the mean gray value of the skin moisture distribution; the smaller the value, the higher the moisture |
| | D28 | -21.24% | Significant difference | 31 (100%) | |
| Skin glossiness (Glossymeter^{®} GL200) (cheek) | D14 | 4.80% | Significant difference | 31 (100%) | The increase in the measurement value indicated an improvement in skin glossiness |
| | D28 | 9.00% | Significant difference | 31 (100%) | |

### (2) Subject self-assessment (satisfaction)

After using the product for 14 days, 94% of the subjects considered deep hydration, 94% of the subjects considered plump and hydrated skin, 94% of the subjects considered good moisturizing effect, 90% of the subjects considered the skin more firm, 87% of the subjects considered the skin more elastic, 87% of the subjects considered wrinkles improved, 87% of the subjects considered fine lines improved, 87% of the subjects considered the overall skin condition improved, 90% of the subjects considered the product suitable for sensitive skin, and 100% of the subjects considered the product mild and non-irritating.

After the product was used for 28 days, 100% of the subjects considered deep hydration, 100% of the subjects considered plump and hydrated skin, 100% of the subjects considered good moisturizing effect, 100% of the subjects considered the skin more firm, 97% of the subjects considered the skin more elastic, 94% of the subjects considered wrinkles improved, 97% of the subjects considered fine lines improved, 90% of the subjects considered the overall skin condition improved, 100% of the subjects considered the product suitable for sensitive skin, 100% of the subjects considered the product mild and non-irritating, 100% of the subjects were satisfied with the overall effect/efficacy of the product, and 100% of the subjects were willing to continue using the product.

### Evaluation conclusion:

Through continuous use of the product for 28 days by 31 healthy Chinese female subjects with sensitive skin, the evaluation results showed that under the evaluation conditions, the emulsion formulation of example 3 had moisturizing, repairing, firming, and anti-wrinkle effects and was suitable for sensitive skin, mild, and non-irritating.

As can be seen from the above:
Through the transdermal permeation efficiency experiment of BSA, it is confirmed that the macromolecular transdermal microemulsion system provided by the present invention can effectively achieve the transdermal delivery of macromolecular substances.

Further, through the animal experiment using botulinum toxin, it is confirmed that the macromolecular transdermal microemulsion system provided by the present invention has a very good transdermal effect for macromolecular substances;
Still further, through the human skin experiment using macromolecular active substances, it is also proved that the macromolecular transdermal microemulsion system provided by the present invention has a very good transdermal effect for macromolecular substances.

It should be noted that the foregoing examples are illustrative only, and the protection of the present invention is not limited to the specific scope of these examples.

## Claims

1. A macromolecular transdermal microemulsion system, comprising an effective amount of a macromolecular active substance capable of exerting a predetermined effect;
wherein
by the mass percentage based on the total weight of the transdermal microemulsion system, the composition of the macromolecular transdermal microemulsion system is:
an oil phase: 7%-36%;
an aqueous phase: 57%-88%;
an emulsifier: 8%-20%.

2. The macromolecular transdermal microemulsion system according to claim 1, wherein the oil phase accounts for 20%-30%, the aqueous phase accounts for 60%-70%, and the emulsifier accounts for 8%-12%; and/or
based on the mass ratio, the composition of the transdermal microemulsion system is:
the oil phase: the aqueous phase: the emulsifier: 2-2.5 : 6-7 : 1.

3. The macromolecular transdermal microemulsion system according to claim 1 or 2, wherein the macromolecular active substance is less than or equal to 300 kDa or less than or equal to 150 kD, and/or
by the mass percentage based on the total weight of the transdermal microemulsion system, the effective amount of the macromolecular active substance in the transdermal microemulsion system is:
the proportion of the macromolecular active substance is less than or equal to 10%, or less than or equal to 5%,
or less than or equal to 2%, or less than or equal to 1%,
preferably, the macromolecular active substance is a biomolecule capable of providing therapeutic, cosmetic, and skin care effects;
another preferably, the macromolecular active substance is water-soluble.

4. The macromolecular transdermal microemulsion system according to any one of claims 1-3, wherein the contained macromolecular active substance is at least selected from any one or more of clostridial neurotoxin, BSA, soluble collagen, and elastin, and/or provided by a yeast extract contained in the system,
preferably, the clostridial neurotoxin is botulinum toxin of type A, B, C, D, E, F, or G, and/or the light chain of the clostridial neurotoxin comprises an amino acid sequence having at least 35%, 50%, 60%, 70%, 75%, 80%, 85%, 90%, 99%, or 100% identity with any one of SEQ ID NOs: 1-7; and/or the heavy chain of the clostridial neurotoxin comprises an amino acid sequence having at least 35%, 50%, 60%, 70%, 75%, 80%, 85%, 90%, 99%, or 100% identity with any one of SEQ ID NOs: 8-14.

5. The macromolecular transdermal microemulsion system according to claim 4, wherein by the mass percentage based on the total weight of the transdermal microemulsion system, in the system:
the effective amount of the clostridial neurotoxin is less than or equal to 0.02%, preferably 0.00001%-0.015%, more preferably 0.0005%-0.015% or 0.0008%-0.0015%;
the effective amounts of the soluble collagen, BSA, and the elastin are each less than or equal to 5%, or less than or equal to 2%, or less than or equal to 1%;
the yeast extract accounts for 1%-10%.

6. The macromolecular transdermal microemulsion system according to any one of claims 1-5, wherein
by the mass percentage based on the total weight of the transdermal microemulsion system, the composition of the system comprises:
5%-20% or 10%-20% or 13%-18% of glycerol; 5%-20% or 5%-15% or 8%-12% of emulsifier, 1%-10% or 3%-8% of butylene glycol or propylene glycol, 0.5%-5% or 1%-3% of laurocapram, 0.3%-3% or 1%-2% of carboxymethyl deacetylated chitin or chitosan derivative, 55%-80% or 60%-70% of water, and the effective amount of the macromolecular active substance.

7. The macromolecular transdermal microemulsion system according to claim 6, wherein the emulsifier is selected from one or more of polysorbate 80, polysorbate 60, polysorbate 20, and sorbitan oleate.

8. The macromolecular transdermal microemulsion system according to any one of claims 1-7, further comprising a preservative,
preferably, by the mass percentage based on the total weight of the transdermal microemulsion system: comprising 0.1%-1% of the preservative based on the mass percentage,
furthermore, the preservative is selected from any one or more of phenoxyethanol, methylparaben, ethylparaben, propylparaben, benzyl alcohol, and sorbic acid.

9. The macromolecular transdermal microemulsion system according to any one of claims 1-8, wherein
the transdermal microemulsion system has an average particle size of 10-15 nm.

10. Use of the macromolecular transdermal microemulsion system according to any one of claims 1-9 in achieving therapeutic, cosmetic, and skincare effects.

11. The use according to claim 10, wherein when the macromolecular transdermal microemulsion system is used, transdermal administration is performed by any one or more of topical application, transdermal instrument, patch application, and spray.

12. Use of the macromolecular transdermal microemulsion system according to any one of claims 1-9 in the manufacture of a medicament or a product for achieving therapeutic, cosmetic, and skincare effects.
